# EUROPEAN PATENT SPECIFICATION

(11) **EP 4 267 964 B1**
(45) Date of publication and mention of the grant of the patent: **03.09.2025**
(21) Application number: 21746464.3
(22) Date of filing: 19.07.2021
(51) Int. Cl.: G01N 33/569

(54) **IN VITRO METHOD FOR PREDICTING MORTALITY IN COVID-19 PATIENTS**
IN-VITRO-VERFAHREN ZUR BESTIMMUNG DER STERBLICHKEIT BEI COVID-19-PATIENTEN
PROCÉDÉ IN VITRO POUR PRÉDIRE LA MORTALITÉ CHEZ DES PATIENTS COVID-19

(30) Priority: 22.12.2020 EP 20383140
(43) Date of publication of application: 01.11.2023
(73) Proprietor: Fundació de Recerca Clínic Barcelona-Institut d'Investigacions Biomèdiques August Pi I Sunyer, 08036 Barcelona (ES); Hospital Clínic de Barcelona, 08036 Barcelona (ES)
(72) Inventor: BERMEJO MARTÍN, Jesús Francisco, 37007 Salamanca (ES); ALMANSA MORA, Raquel, 37007 Salamanca (ES); SANTOS TEDIM SOUSA PEDROSA, Ana Sofía, 37007 Salamanca (ES); BUSTAMANTE MUNGUIRA, Elena, 47005 Valladolid (ES); TAMAYO LOMAS, Luis Mariano, 47012 Valladolid (ES); ALDECOA ÁLVAREZ- SANTULLANO, César, 47012 Valladolid (ES); DOMINGUEZ-GIL GONZALEZ, Marta, 47012 Valladolid (ES); EIROS BOUZA, Jose María, 47012 Valladolid (ES); BARBÉ ILLA, Ferrán, 25198 Lleida (ES); TORRES MARTÍ, Antoni, 08036 Barcelona (ES); MICHELOUD GIMÉNEZ, Dariela Edhit, 28007 Madrid (ES); GOMEZ GARCÍA, Jose Manuel, 28007 Madrid (ES); GONZÁLEZ RIVERA, Milagros, 28007 Madrid (ES); LOPEZ IZQUIERDO, Raul, 47007 Valladolid (ES); KELVIN, David Joseph, Halifax, Nova Scotia, Nova Scotia B3N 3B9 (CA)
(74) Representative: Hoffmann Eitle
(86) International application number: PCT/EP2021/070131
(87) International publication number: WO 2022/135752

(56) References cited:
- LE HINGRAT QUENTIN ET AL: "SARS-CoV-2 N-antigenemia: A new COVID-19 marker and a potential alternative to nucleic acid amplification techniques", MEDRXIV, 15 September 2020 (2020-09-15), pages 1 - 7, XP055799960, Retrieved from the Internet <URL:https://www.medrxiv.org/content/10.1101/2020.09.14.20191759v1.full.pdf> [retrieved on 20210429], DOI: 10.1101/2020.09.14.20191759
- MANUS JEAN-MARIE: "Covid-Quantigene , sur la piste du Sars-CoV-2", REVUE FRANCOPHONE DES LABORATOIRES, ELSEVIER, AMSTERDAM, NL, vol. 2020, no. 526, 31 October 2020 (2020-10-31), pages 5, XP086330906, ISSN: 1773-035X, [retrieved on 20201031], DOI: 10.1016/S1773-035X(20)30280-X
- OGATA ALANA F ET AL: "Ultra-Sensitive Serial Profiling of SARS-CoV-2 Antigensand Antibodies in Plasma to Understand DiseaseProgression in COVID-19 Patients with Severe Disease", vol. 66, no. 12, 1 December 2020 (2020-12-01), US, pages 1562 - 1572, XP055799823, ISSN: 0009-9147, Retrieved from the Internet <URL:https://watermark.silverchair.com/hvaa213.pdf?token=AQECAHi208BE49Ooan9kkhW_Ercy7Dm3ZL_9Cf3qfKAc485ysgAAAsMwggK_BgkqhkiG9w0BBwagggKwMIICrAIBADCCAqUGCSqGSIb3DQEHATAeBglghkgBZQMEAS4wEQQM01qCpDhfOgDU-LfJAgEQgIICdkZrlEXrm6gRwMH_MGT19JwY3idHdJfPR2Ho6wHxWpaIS7Fxii9hKUa7MOYdgBcf_OfugixxGem8tGv2WMQT5MWERBwR> DOI: 10.1093/clinchem/hvaa213
- BERMEJO-MARTIN JESÚS F ET AL: "Viral RNA load in plasma is associated with critical illness and a dysregulated host response in COVID-19", 14 December 2020 (2020-12-14), pages 1 - 13, XP055799702, Retrieved from the Internet <URL:https://link.springer.com/content/pdf/10.1186/s13054-020-03398-0.pdf> [retrieved on 20210429], DOI: 10.1186/s13054-020-03398-0
- INSTIATY ET AL: "Antiviral treatment of COVID-19: a clinical pharmacology narrative review", vol. 29, no. 3, 18 July 2020 (2020-07-18), pages 332 - 45, XP055800105, ISSN: 0853-1773, Retrieved from the Internet <URL:https://mji.ui.ac.id/journal/index.php/mji/article/download/4652/1984> DOI: 10.13181/mji.rev.204652

## Description

### FIELD OF THE INVENTION

The present invention refers to the medical field. Particularly, it refers to the *in vitro* use of a SARS-CoV-2 antigen nucleocapsid (N), measured in plasma, serum or blood samples obtained from the patient, for the prognosis of mortality risk of patients suffering from SARS-CoV-2 infection, for predicting the response of patients suffering from SARS-CoV-2 infection to an antiviral therapy, or for selecting patients suffering from SARS-CoV-2 infection for receiving an antiviral therapy.

### STATE OF THE ART

A week after alerting the WHO of a cluster of pneumonia of unknown etiology in Wuhan, the Chinese authorities announced on 7 January 2020 that a novel coronavirus was identified as the cause of these pneumonia. According to phylogenetic analysis, this novel severe acute respiratory syndrome coronavirus 2 (SARS-CoV-2), previously named 2019-nCoV, belongs to the B lineage of Betacoronavirus genus and the Sarbecovirus subgenus and has more than 85% nucleotide sequence identity with a bat SARS-like CoV genome published previously. On January 30, WHO declared COVID-19 outbreak a public health emergency of international concern and the disease has now spread worldwide.

In this context, highly sensitive and specific tests are crucial to identify and manage COVID-19 patients and implement control measures to limit the outbreak. Real time reverse transcription polymerase chain reaction (RT-qPCR) in respiratory samples is the current recommended laboratory method to diagnose SARS-CoV-2 acute infection. However, performing RT-qPCR requires special equipment and skilled laboratory personnel familiar with molecular techniques. Moreover, molecular tests are costly and often time consuming.

Consequently, test designed to detect SARS-CoV-2 antigen in nasopharyngeal secretions have been developed and have been included in the first line of diagnostic tests for COVID-19.

However, there is still an unmet medical need of finding highly sensitive and specific tests aimed not only at diagnosing SARS-CoV-2 infection but also being able of predicting mortality risk, determining the presence of viremia and/or high degree of viral replication. This type of test would bring the opportunity of rapidly identifying and selecting patients with a higher mortality risk for receiving an antiviral therapy. In fact, critically ill patients, who could benefit the most from antiviral treatment, would be identified at an early state and treated accordingly.

The present invention is focused on solving the above indicated problem, and a test designed to detect SARS-CoV-2 antigen is herein provided, which can be used to predict mortality risk or for predicting the response of patients suffering from SARS-CoV-2 infection to an antiviral therapy.

Le Hingrat Q. et al, medRxiv, 15.09.2020, assesses the performance of an ELISA microplate assay quantifying SARS-CoV-2 nucleocapsid antigen.

Bermejo-Martín J. et al, 14.12.2020, teaches that the presence of SARS-CoV-2 RNA in plasma is associated with critical illness in patients with COVID-19.

### DESCRIPTION OF THE INVENTION

### Brief description of the invention

As explained above, the present invention refers to the *in vitro* use of a SARS-CoV-2 antigen nucleocapsid (N), measured in plasma, serum or blood samples obtained from the patient, for the prognosis of mortality risk of patients suffering from SARS-CoV-2 infection, for predicting the response of patients suffering from SARS-CoV-2 infection to an antiviral therapy, or for selecting patients suffering from SARS-CoV-2 infection for receiving an antiviral therapy.

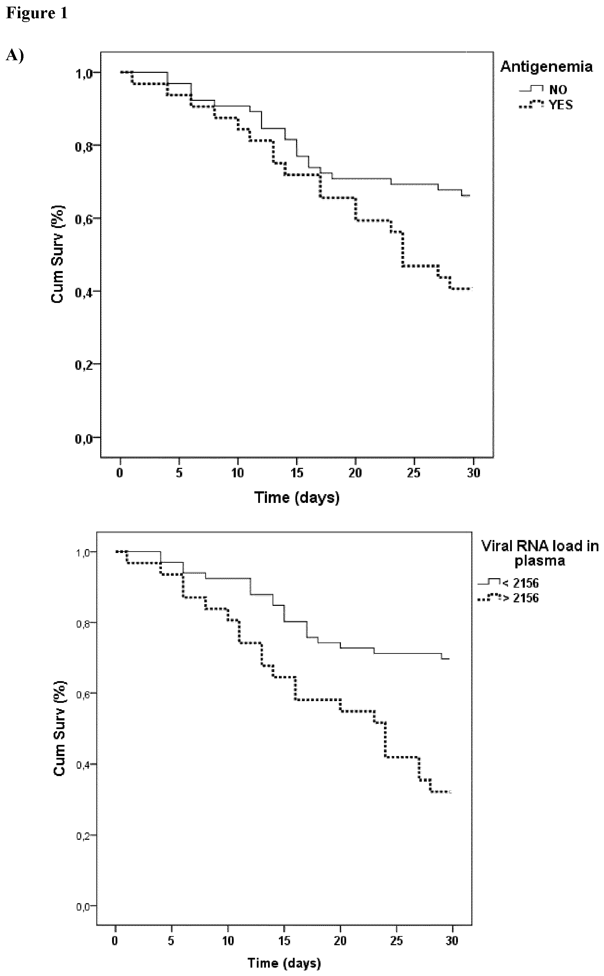

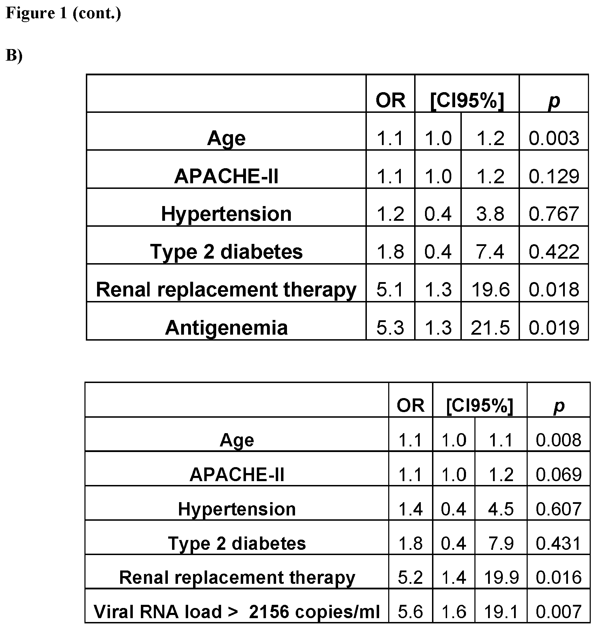

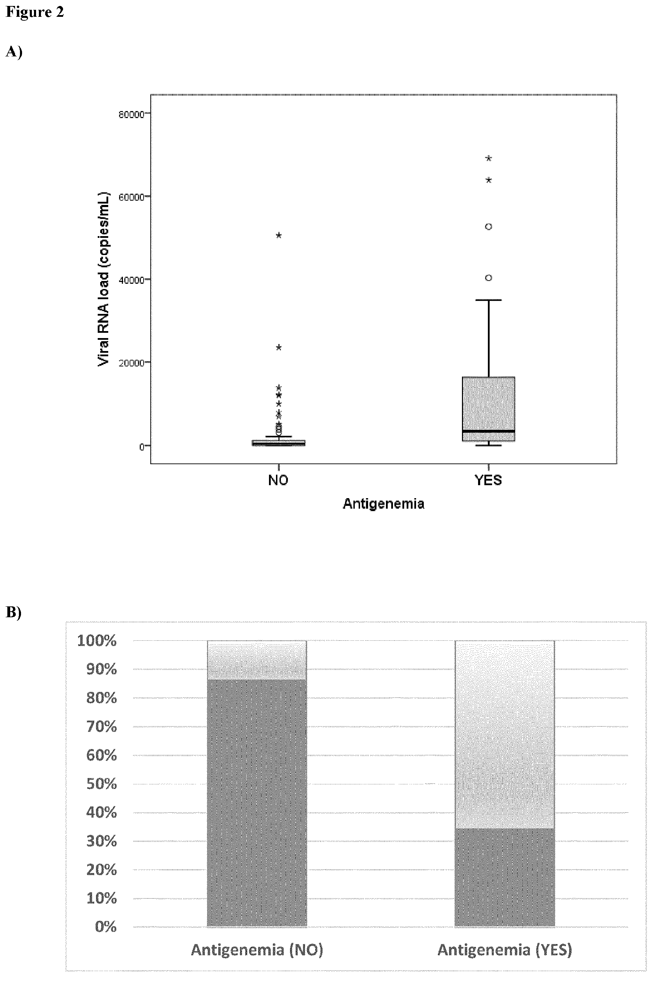

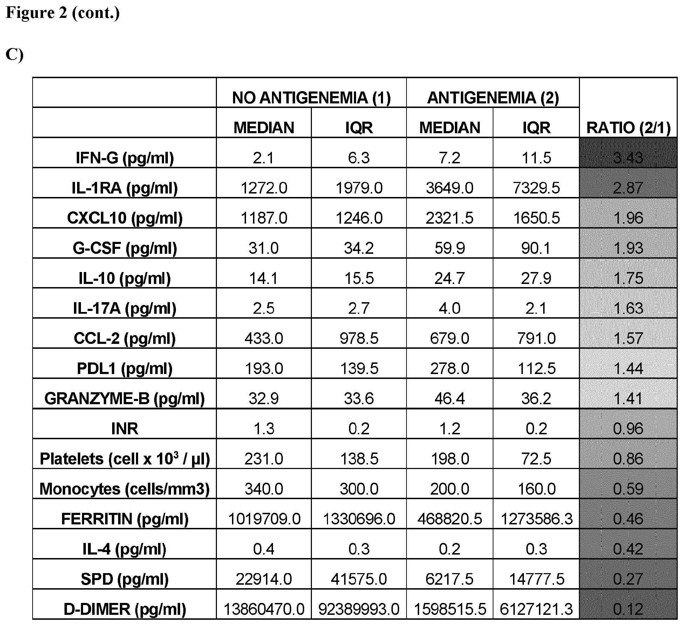

In the discovery phase of this study, wherein a discovery cohort of critically ill hospitalised patients were analysed (see **Example 1, Table 1,** **Figure 1** and **Figure 2**), we employed a rapid test to detect SARS-CoV-2 antigen (Panbio COVID-19 rapid test device, Abbott, Illinois, United States) in plasma from critically ill COVID-19 patients, collected in the first 24 hours following admission to the ICU. 100 ul of plasma were diluted with 100 ul of the test buffer and loaded into the test device. Fifteen minutes later, the test result was read as positive or negative for the presence of antigen nucleocapsid (N). As showed by the Kaplan Meier curves, patients with viral antigen nucleocapsid (N) in plasma died earlier than those with no antigen nucleocapsid (N) in plasma (**Figure 1**). In the multivariate logistic regression analysis, the presence of viral antigen nucleocapsid (N) in plasma translated into a 5-fold risk of dying in the first 30 days following admission to the ICU (**Figure 1**). A similar odds ratio was found for the presence of viral RNA load in plasma higher than 2156 copies/ml (**Figure 1**). The reason why antigenemia is associated to poor outcome is unclear, but further evidence suggests that patients who present viral antigen nucleocapsid (N) in blood are not able to control viral replication: 1) patients with antigenemia showed higher viral RNA loads in plasma than those with no antigenemia (as quantified by using droplet digital PCR) (**Figure 2**); 2) patients with antigenemia showed a lower frequency of anti-SARS-CoV-2 IgG (**Figure 2**); 3) these patients showed in addition higher levels of chemokines increasing in the context of active viral replication (CXCL10, CCL2), of molecules inducing immunosuppression (IL-1RA, IL-10 and PD-L1), lower levels of the antiviral molecule SPD, lower monocyte counts, and of the antibody inducer cytokine IL-4 (**Figure 2**). Finally, patients with antigenemia showed lower concentration of ferritin in plasma, and lower D-dimers and INR, with (paradoxically), lower platelet counts (**Figure 2**), findings which biological implications remains to be elucidated.

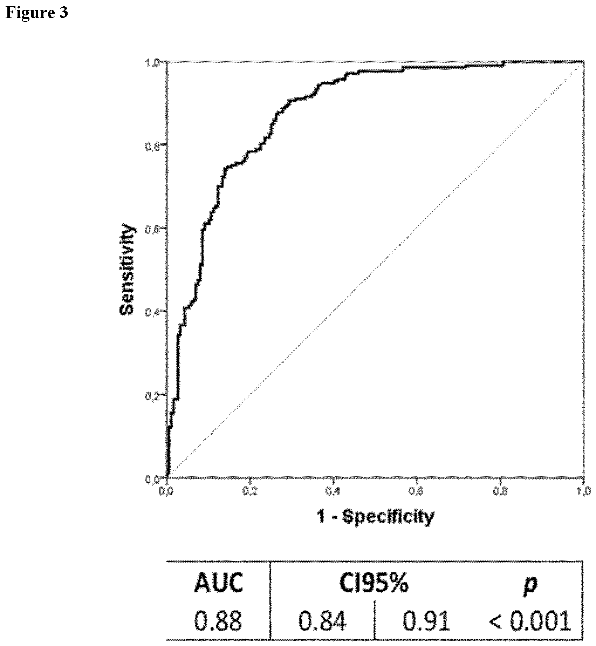

Our results demonstrate that detecting the presence of viral antigen nucleocapsid (N) in plasma using these fast tests informs on the probability of death of COVID-19 patients, making these tests a valuable tool to better select those patients to be admitted to the ICU, and helping to individualise treatment. Our results demonstrate also that the presence of viral antigen nucleocapsid (N) in plasma is a surrogated marker of increased viral replication, which could help to identify those patients deserving treatments with antiviral activity (drugs, monoclonal antibodies, hyperimmune serum i.e). Moreover, during the validation phase, wherein a validation cohort of non-critically-ill hospitalised patients were analysed (see **Example 2, Table 2, Table 3, Table 4,** **Figure 3****,** **Figure 4** and **Figure 5**), a cohort of non-critically-ill patients suffering from SARS-CoV-2 infection, was analysed. More specifically, we evaluated the prevalence of sepsis secondary to SARS-CoV-2 infection in a cohort of 400 patients suffering from SARS-CoV-2 infection at hospitalization. We next evaluated whether the presence of SARS-CoV-2 antigen nucleocapsid (N) in plasma is associated to sepsis at hospital admission. Finally, we studied if antigenemia predicts 90-day mortality in these patients. Such as it is shown below, this validation phase evidences that sepsis is present at hospitalization in a large proportion (54%) of non-critically ill patients suffering from SARS-CoV-2 infection. Sepsis secondary to SARS-CoV-2 infection affected mostly to the respiratory, renal and coagulation function and translated into a higher mortality 90 days following hospitalization.

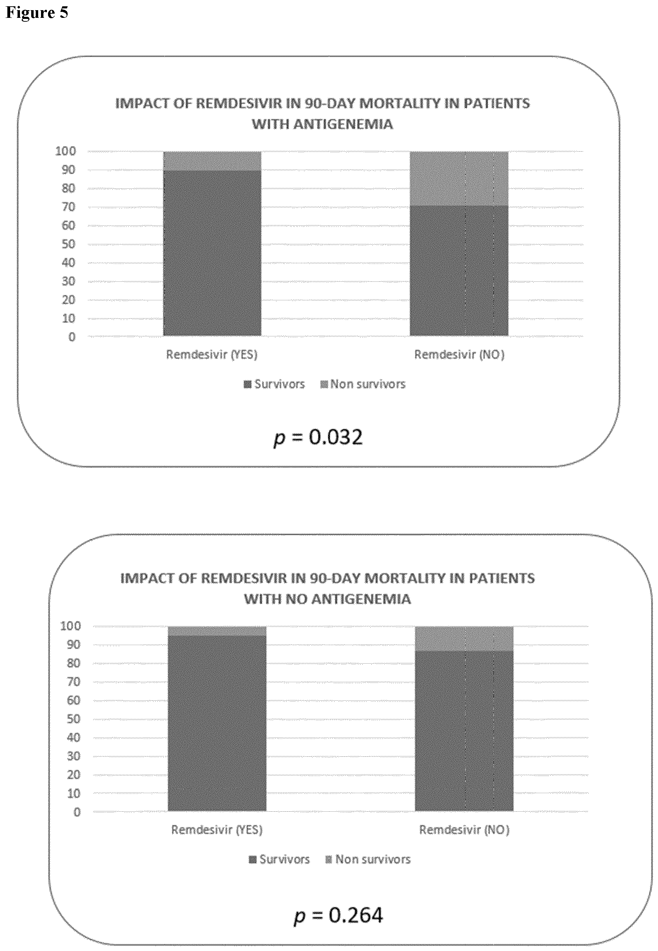

Finally, it is important to note that during the validation phase, the response of patients showing antigenemia to specific antiviral therapy or treatment was also assessed (see **Figure 5**). Particularly, in those patients of our cohort showing antigenemia, remdesivir treatment translated into a lower frequency of death at 90 days following hospitalization compared with patients not receiving this drug. In contrast, remdesivir had no impact on 90-day mortality in patients with no antigenemia. Thus, antigenemia could help also to identify those patients potentially receiving benefit from antiviral therapies like Broad-spectrum antivirals (BSAs), antibodies or even hemofilters aimed at clearing either whole virus of viral proteins from the blood.

So, the first embodiment of the present invention refers to an *in vitro* method for the prognosis of mortality risk in patients suffering from SARS-CoV-2 infection, which comprises determining the presence or the level of SARS-CoV-2 antigen nucleocapsid (N) in plasma, serum or blood samples obtained from the patient, wherein the determination of the presence of a SARS-CoV-2 antigen nucleocapsid (N), or the quantification of a level of a SARS-CoV-2 antigen nucleocapsid (N) statistically higher as compared with a pre-established threshold value (this would be the value measured in mildly infected patients which have not been hospitalized), is an indication of bad prognosis and/or of mortality risk.

In a preferred embodiment, the patient is suffering from sepsis secondary to SARS-CoV-2 infection. The second embodiment of the present invention refers to an *in vitro* method for predicting the response of patients suffering from SARS-CoV-2 infection to an antiviral therapy with remdesivir which comprises assessing the presence or the level of a SARS-CoV-2 antigen nucleocapsid (N) in plasma, serum or blood samples obtained from the patient, wherein if the presence of SARS-CoV-2 antigen nucleocapsid (N) is identified, or a statistically higher level of SARS-CoV-2 antigen nucleocapsid (N) is quantified as compared with a pre-established threshold value, this is an indication that the patient may respond to the antiviral therapy.

The third embodiment of the present invention refers to an *in vitro* method for selecting patients suffering from SARS-CoV-2 infection for receiving an antiviral remdesivir therapy, which comprises determining the presence or the level of SARS-CoV-2 antigen nucleocapsid (N) in plasma, serum or blood samples obtained from the patient, wherein if the presence of SARS-CoV-2 antigen nucleocapsid (N) is determined, or a statistically higher level of SARS-CoV-2 antigen nucleocapsid (N) is quantified as compared with a pre-established threshold value, the patient is elected for receiving an antiviral therapy.

The fourth embodiment of the present invention refers to the *in vitro* use of a SARS-CoV-2 antigen nucleocapsid (N) derived from plasma, serum or blood samples obtained from the patient, for the prognosis of mortality risk of patients suffering from SARS-CoV-2 infection, for predicting the response of patients suffering from SARS-CoV-2 infection to an antiviral remdesivir therapy, or for selecting patients suffering from SARS-CoV-2 infection for receiving an antiviral remdesivir therapy.

In a preferred embodiment, the above methods are characterized in that the presence or the level of the antigen nucleocapsid (N) is measured by using mass spectrometry or an immunoassay, preferably ELISA, immunochromatography, nephelometry, Luminex, SimplePlex, or any other technique based on the antigen-antibody reaction, and also those based on recognition of SARS-CoV-2 antigens by DNA or RNA molecules such as those using aptamers or oligonucleotide-labelled antibodies.

Disclosed herein is also a kit-of-parts for performing any of the methods described above consisting of:
a. Means for obtaining plasma, serum or blood samples from the patient.
b. Antibodies which specifically bind antigen nucleocapsid (N) of the SARS-CoV-2.

In a preferred embodiment the kit-of-parts is an immunoassay, preferably ELISA, immunochromatography, nephelometry, Luminex, SimplePlex, or any other technique based on the antigen-antibody reaction, and also those based on recognition of SARS-CoV-2 antigens by DNA or RNA molecules such as those using aptamers or oligonucleotide-labelled antibodies.

Finally, described herein is also an *in vitro* method for the diagnosis or prognosis of sepsis secondary to SARS-CoV-2 infection, which comprises determining the presence or the level of SARS-CoV-2 antigen nucleocapsid (N) in plasma, serum or blood samples obtained from the patient, wherein the determination of the presence of a SARS-CoV-2 antigen nucleocapsid (N), or the quantification of a level of a SARS-CoV-2 antigen nucleocapsid (N) statistically higher as compared with a pre-established threshold value (this would be the value measured in mildly infected patients which have not been hospitalized), is an indication that the patient is suffering or will suffer from sepsis secondary to SARS-CoV-2 infection.

In a preferred embodiment, the present invention is a computer-implemented invention, wherein a processing unit (hardware) and a software are configured to:
- Receive information/data about the presence or concentration level SARS-CoV-2 antigen nucleocapsid (N) in blood, plasma or serum samples obtained from the patient,
- Process the information/data received for finding substantial variations or deviations, and
- Provide an output through a terminal display of the variation or deviation found, wherein a variation or deviation indicates that the subject has a bad prognosis and/or mortality risk.

The present disclosure also refers to a computer program or a computer-readable media containing means for carrying out the method described above.

For the purpose of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of". Thus, the phrase "consisting of" indicates that the listed elements are required or mandatory, and that no other elements may be present.
- By "therapeutically effective dose or amount" is intended an amount that, when administered as described herein, brings about a positive therapeutic response in a subject suffering a SARS-CoV-2 infection. The exact amount required will vary from subject to subject, depending on the age, and general condition of the subject, the severity of the condition being treated, mode of administration, and the like. An appropriate "effective" amount in any individual case may be determined by one of ordinary skill in the art using routine experimentation, based upon the information provided herein.
- A reference control level is herein mentioned, when referring to the level of the antigens observed in patients. The patient is likely to have a bad prognosis with a given sensitivity and specificity if the levels of the antigens in the patient are above said reference control level. A reference value can be a "threshold value" or a "cut-off" value. Typically, a "threshold value" or "cut-off value" can be determined experimentally, empirically, or theoretically. According to the present invention, the "threshold" value refers the level of antigens identified in mild infected patients which has not been hospitalized or have a good prognosis.
- In the context of the present invention the term "antigenemia" refers to the condition of having an antigen present in plasma, serum or blood samples. Particularly, the antigen is a structural protein of the SARS-CoV-2 selected from: nucleocapsid (N), Spike (S), Membrane (M) and/or Envelope (E) proteins, preferably the nucleocapsid (N) protein of SARS-CoV-2, most preferably nucleocapsid (N).

### Brief description of the figures

**Figure 1****. A)** Kaplan Meier curves for 30-day mortality. **B)** Multivariate logistic regression analysis for 30-day mortality. Discovery cohort > critically ill hospitalised patients.
**Figure 2****. A)** Viral RNA load in plasma (p<0.001). **B)** % of patients with anti-SARS-CoV-2 IgG (dark blue) (*p*<0.001). **C)** Laboratory parameters in patients with presence / absence of antigenemia (p<0.05). Discovery cohort > critically ill hospitalised patients.
**Figure 3****.** Diagnostic accuracy of the COV-QUANTO^{®} ELISA to detect N-antigenemia as profiled by the PANBIO^{®} COVID-19 Ag test by AUC analysis. Validation cohort > non-critically ill hospitalised patients.
**Figure 4****.** Impact of N-antigenemia on survival. Validation cohort > non-critically ill hospitalised patients.
**Figure 5****.** Impact of the antiviral remdesivir in 90-day mortality in patients with antigenemia vs patients without antigenemia. Validation cohort > non-critically ill hospitalised patients.

### Detailed description of the invention

The present invention is illustrated by means of the Examples set below without the intention of limiting its scope of protection.

### Example 1. Discovery phase wherein a discovery cohort of critically ill hospitalised patients were analysed (Table 1, Figure 1 and Figure 2)

### Example 1.1. Patient characteristics

**Table 1** included below describes the cohort of critically ill patients suffering from COVID-19 who have been analysed in the present invention for the study of antigenemia and risk of mortality at 30 days.

### Example 1.2. Viral load analyses

By using the Bio-Rad SARS-CoV-2 ddPCR kit (digital PCR), the best cut-off point for determining viral load has been established, by analysing the N1 fragment of the N gene of the virus. 1233 copies/mL in plasma of cDNA of the N1 fragment of the virus yield a sensitivity of 75% and a specificity of 75% to detect the presence of antigenemia. In other words, high levels viral load (or viral replication) are identified, with a sensitivity of 75% and a specificity of 75%, when more than 1233 copies of cDNA of the N1 fragment / mL are identified.

### Example 1.3. Antigenemia in plasma samples translates into an earlier morality

Such as it is shown in **Figure 1** (**A,** upper) the present invention provides evidence that the presence of antigenemia in plasma samples translates into an earlier mortality, as assessed by the Kaplan Meier curves. This way, non-survivors showing antigenemia died 2 days earlier than those with no antigenemia. In addition, multivariate logistic regression analysis evidence that antigenemia was a predictor of mortality at 30 days following admission to the ICU, independently of major confounding variables **Figure 1** (**B,** upper). In turn, the presence of high levels of viral RNA in plasma **Figure 1** (A, lower), also translated into an earlier mortality, and constituted an independent risk factor for mortality, with a similar odds ratio than that of antigenemia **Figure 1** (**B,** lower). While quantifying viral load requires of complicated PCR-based methods, the methods described in the present invention to determine the presence of antigenemia are easier and faster to perform, since they are based in the antigen-antibody reaction.

### Example 1.4. Patients with antigenemia showed higher viral loads in plasma

Such as it is shown in **Figure 2A****),** patients with antigenemia showed higher viral loads in plasma, as assessed by using droplet digital PCR. As shown in **Figure 2B****),** patients with antigenemia showed a lower frequency of specific IgG anti SARS-CoV-2, as assessed by the Abbott Architect SARS-CoV-2 IgG Assay (Illinois, U.S.A). According to **Figure 2C****),** patients with antigenemia showed higher levels of several laboratory parameters indicating dysregulation of the host response to infection (indicated with red colour in the "ratio" column), and lower levels of other parameters indicating also dysregulation of the host response to infection (indicated with blue colour in the "ratio" column).

### Example 2. Validation phase wherein a validation cohort of non-critically-ill hospitalised patients were analysed (Table 2, Table 3, Table 4, Figure 3, Figure 4 and Figure 5)

### Example 2.1. Study design

400 adult patients with a positive nasopharyngeal swab polymerase chain reaction (PCR) or antigen test for SARS-CoV-2 performed at participating hospitals were recruited at hospitalization at the ward from June 13^{th}, 2020 to February 14^{th,} 2021. 216 patients were recruited at the "Hospital Universitario Río Hortega" (Valladolid, Spain), and 184 at "Hospital Arnau de Vilanova and Santa Maria", Lérida, Spain at the Internal Medicine and Pneumology Services. Patients admitted directly to the ICU from the emergency department, with no prior hospitalization at the ward, were excluded from this study. This was a sub-study of the CIBERES-UCI-COVID project, registered at Clinicaltrials.gov with the identification NCT04457505. De-identified patient data were collected and stored via the REDCap electronic data capture tool, hosted at the Centro de Investigación Biomédica en Red (CIBER), Spain. Data from patients' medical records were incorporated into a separate database by trained local researchers.

### Example 2.2. Clinical definitions

acute respiratory distress syndrome (ARDS) was identified by using the criteria described in the Berlin definition. Sepsis was identified by using the criteria proposed by the SEPSIS-3 consensus.

### Example 2.3. Ethics

Written or oral informed consent was obtained directly from all patients, or their legal representative, before enrolment. Scientific and ethical approval of the study protocol was obtained from the respective scientific committees for clinical research of the participant hospitals.

### Example 2.4. Antigenemia profiling in plasma

Plasma from blood collected in EDTA tubes samples was obtained in the first 24 hours following patients' admission to the ward and stored at -80°C until analysis. The presence/absence of N-antigen of SARS-CoV-2 in plasma was evaluated by using the Panbio^{®} COVID-19 Ag Rapid Test Device from Abbott (Chicago, IL, USA). The concentration of N antigen in the same plasma sample was assessed by using the COV-QUANTO^{®} ELISA (AAZ), according to manufacturer's recommendations.

### Example 2.4. Statistical analysis

Statistical analysis was performed using IBM SPSS Statistics 25.0 (SPSS INC, Armonk, NY, U.S.A). The level of significance was set at 0.05 (2-tailed). For descriptive analysis of patient characteristics, the differences between patients with presence or absence of antigenemia were assessed using the Chi-square test or Fisher's Exact Test for categorical variables. Differences for continuous variables were evaluated by using the Mann-Whitney U test. The accuracy of N-antigen concentration in plasma measured by the COV-QUANTO^{®} test to detect the presence of antigenemia as assessed by the Panbio^{®} COVID-19 Ag Rapid Test was studied by calculating the area under the receiver operating characteristic curve (AUROC). AUROC was also employed to evaluate the accuracy of N-antigen concentration for distinguishing between presence and absence of sepsis at hospital admission and also between survivors and non survivors 90 days following hospitalization (Supp file 1). The cut-off yielding the best balance between sensitivity and specificity was obtained by calculating the optimal operating point (OOP) in the AUROC, namely, the point on the AUROC that had the minimum distance to the upper left corner calculated by Pythagorean theorem. According to presence/absence and OOP for the AUROC curve, antigen levels were stratified into categorical variables which were evaluated for their association with the presence of sepsis at hospital admission and also with the risk of 90-day mortality by using multivariate logistic regression analysis. Variables from **Table 2** which were associated with the presence of sepsis or with 90-day mortality at the level *p* < 0.1 in the univariate analysis were introduced as adjusting variables in the respective multivariate one. Survival in the first 90 days was represented by using Kaplan-Meier curves and groups were compared using the log-rank test.

### Example 2.5. Clinical characteristics of the patients (Table 2)

Patients were mostly elderly individuals (median age of 72 years) with moderate Charlson comorbidity index (CCI) (median CCI of 4 points). The most frequent comorbidities were hypertension, dyslipidemia, diabetes, obesity and central nervous system disease. At hospital admission, half of the patients presented with asthenia and cough, and 60% had dyspnea, with 62% showing bilateral involvement in the chest X-ray. Median SOFA score was 2 points. 54% of the patients presented with sepsis. The most frequent failure was that affecting the respiratory function (62%), followed by that involving coagulation (28%) and by that affecting renal function (18%). The prevalence of other kind of failure (cardiovascular, CNS and liver) was below 7%. Patients showed moderate inflammation as evidenced by the C reactive protein levels, increased levels of LDH denoting tissue destruction, and moderate lymphopenia. Patients stayed 8 days in median at the hospital. 9% of them needed of non-invasive mechanical ventilation, 20.7% of high-flow oxygen therapy, 9.7% were transferred to the ICU, with 5.7% needing of invasive mechanical ventilation. Most common treatments were antibiotics, steroids and tocilizumab. ARDS was the most frequent complication, affecting to 35% of the patients. 19.5% of the patients had died by day 90 following hospital admission.

53% of the patients showed the presence of N-antigen in plasma, as assessed by the PANBIO^{®} COVID-19 Ag test. The profile of age, sex and comorbidities was similar between those patients with presence or absence of antigenemia, except chronic heart failure and asthma which were more frequently found in those patients with antigenemia. No differences were neither found regarding days since the onset of the symptoms between those patients with presence or absence of antigenemia (4 days vs 5 days respectively). Patients with antigenemia showed higher levels of CRP, LDH, creatinine and lower concentrations of lymphocytes, monocytes and platelets in blood. The prevalence of sepsis was higher in the group of patients with antigenemia as compared to those with no antigenemia (61% vs 45.7%). Patients with sepsis showed an increased 90-day mortality compared with those with no sepsis (26.3% vs 11.3%) (*p* < 0.001).

Specifically, patients with antigenemia showed more frequently failure at the respiratory function and coagulation. Patients with antigenemia showed higher CURB-65 and MULBTSA scores. These patients needed more frequently of respiratory support with low-flow oxygen therapy, non-invasive mechanical ventilation and high-flow oxygen therapy and invasive mechanical ventilation. They stayed longer at the hospital, were more often transferred to the ICU, needed more frequently of invasive mechanical ventilation, and had an increased incidence of ARDS. Finally, patients with antigenemia had an increased mortality compared to those with no antigenemia (25.8% vs 12.3%).

Levels of N-antigen in plasma assessed by the COV-QUANTO^{®} ELISA test showed an excellent accuracy to detect the presence of antigenemia assessed by the PANBIO^{®} COVID-19 Ag test (AUC 0.88, **Figure 3**). N-antigen levels were higher in those patients with sepsis compared to those with no sepsis (1612 [2902] vs 369 [1874]) (median in pg/mL, [IQR]), and also in those patients who died in the first 90 days following admission to the hospital (2113 [3064] vs 947 [2491]) (median in pg/mL, [IQR]).

### Example 2.6. Antigenemia as risk factor of sepsis (Table 3):

Chronic renal failure was the factor more strongly associated with the development of sepsis due to COVID-19, followed by COPD, active neoplasia and male sex. Antigenemia was an independent risk factor of sepsis, conferring a 1.6 / 2.2-fold increase in the risk of sepsis as assessed by the PANBIO^{®} COVID-19 Ag test and the COV-QUANTO^{®} ELISA test respectively.

### Example 2.7. Antigenemia as predictor of 90-day mortality (Table 4):

Need of mechanical ventilation was the strongest predictor of death, followed by the antecedent of chronic disease of the CNS, the SOFA score and age. Antigenemia was an independent risk factor of 90-day mortality, conferring a 2.2 / 1.9-fold increase in the risk of death as assessed by the PANBIO^{®} COVID-19 Ag test and the COV-QUANTO^{®} ELISA test respectively.

### Example 2.9. Antigenemia and survival mean time

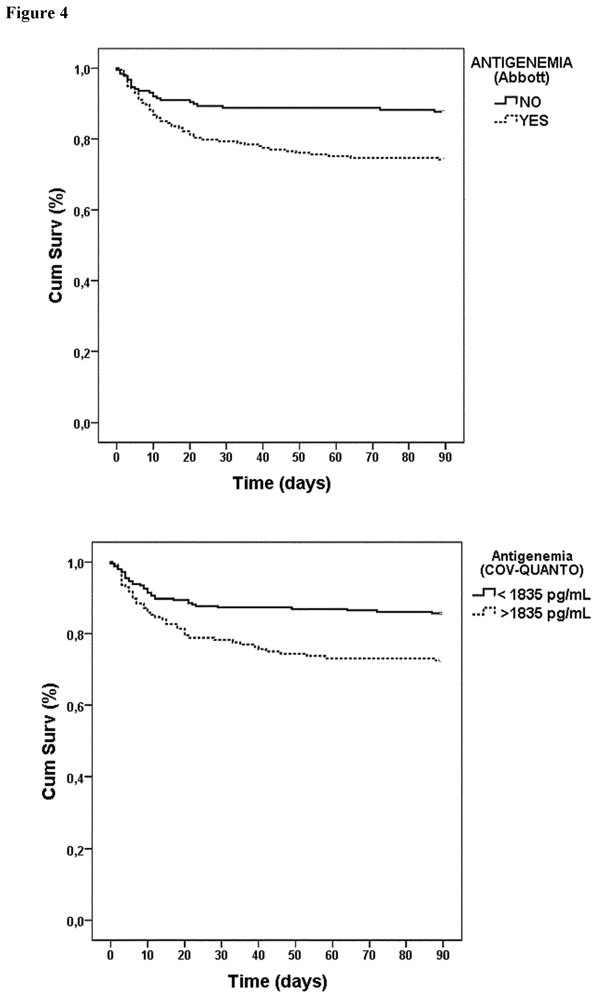

The presence of antigenemia was associated with a reduction in the survival mean time in the 90 first days following hospitalization of 9.42 days and 9.33 days based on the PANBIO^{®} COVID-19 Ag test and the COV-QUANTO^{®} ELISA test respectively (**Figure 4**).

### Example 2.10. Antiviral therapy in patients showing antigenemia vs patients with no antigenemia

In those patients of our cohort showing antigenemia, remdesivir treatment translated into a lower frequency of death at 90 days following hospitalization:10% (3 out of 30 patients), compared with 29 % (52 out of 182) in patients not receiving this drug (p = 0.032). In contrast, remdesivir had no impact on 90-day mortality in patients with no antigenemia: 5% (1 out of 21) in the remdesivir group vs 13% (22 out of 166) in the group not receiving remdesivir (p = 0.264) (see **Figure 5**). Antigenemia could help also to identify those patients potentially receiving benefit from therapies with haemofilters aimed at clearing either whole virus of viral proteins from the blood.

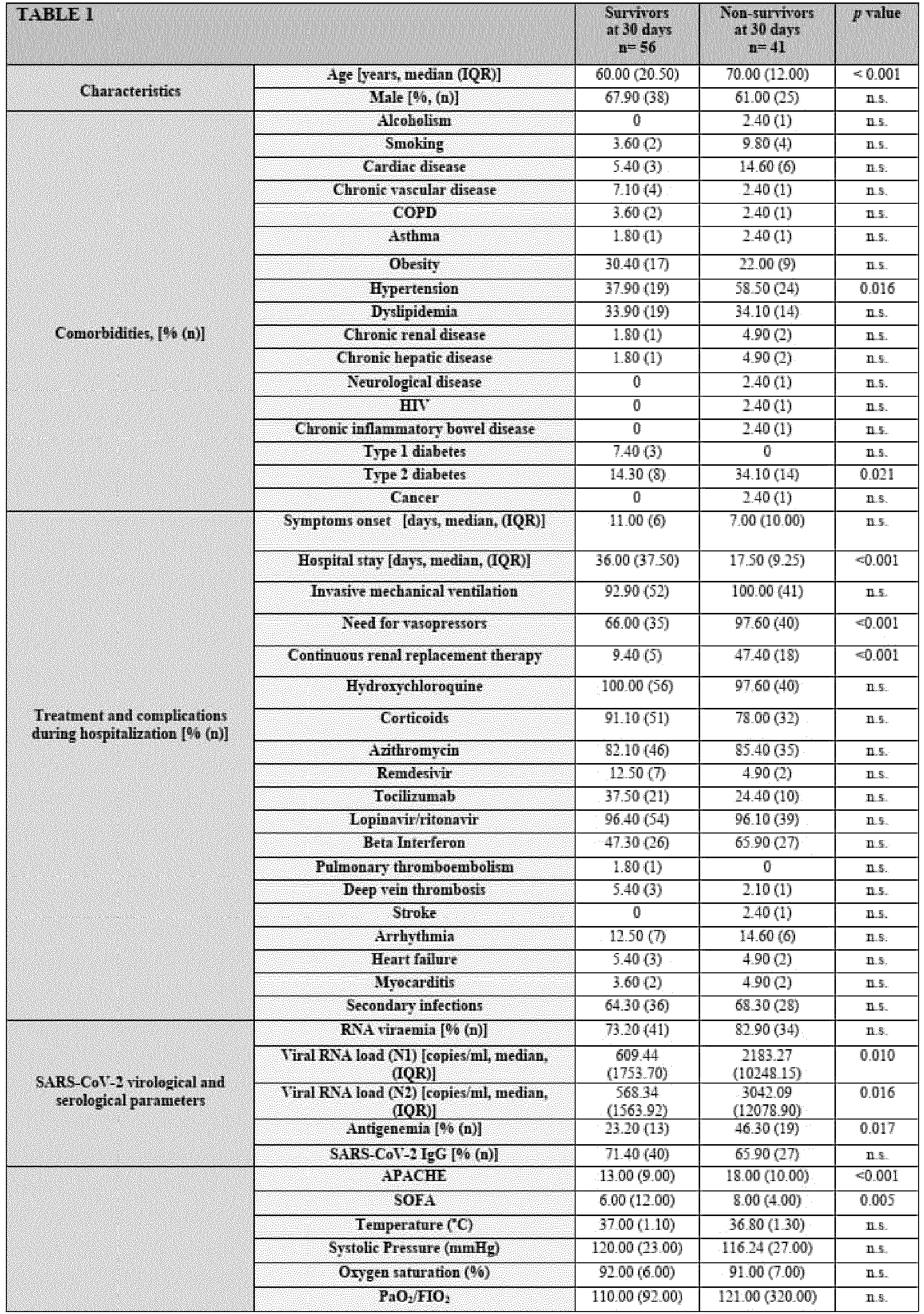

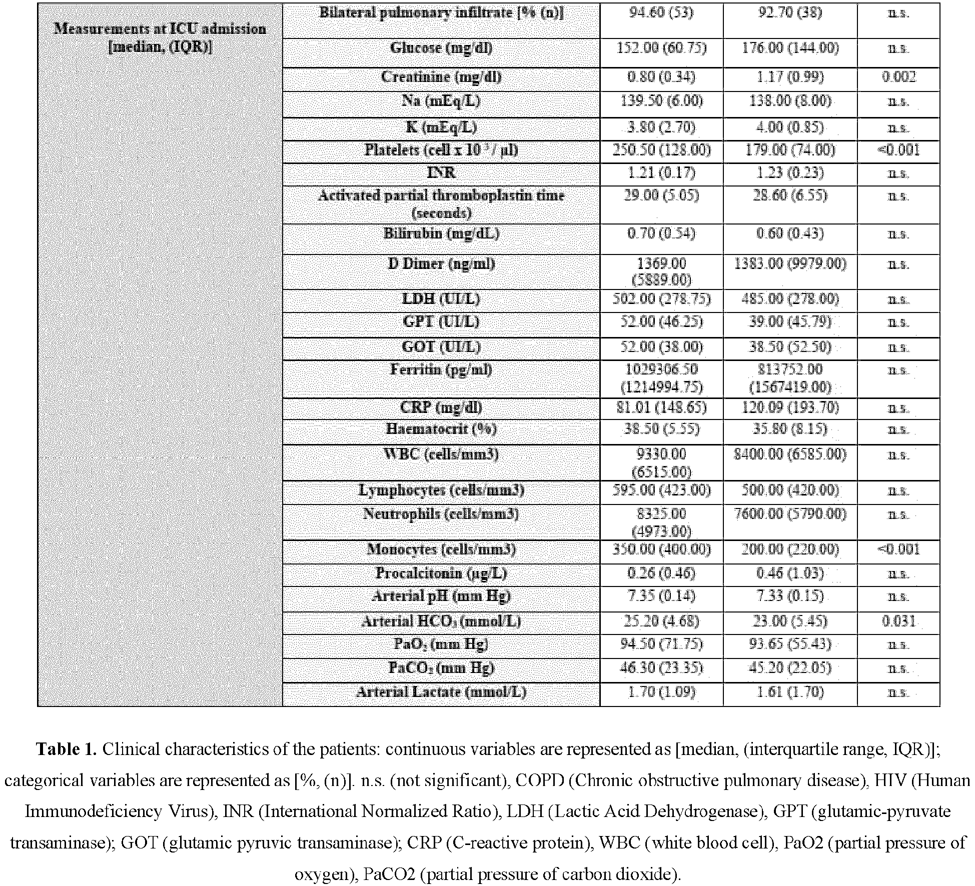

### Table 2

**Table 2. Clinical characteristics of patients. Statistics: Continuous variables are represented as median (interquartile range) and categorical variables as absolute count (%). P-values were calculated by Mann-Whitney for continuous variables and chi-squared tests for categorical variables. Significant differences are shown in bold. Abbreviations: p-value, level of significance; COPD, chronic obstructive pulmonary disease; BUN, Blood Urea Nitrogen; GPT, glutamic-pyruvate transaminase; LDH, lactic acid dehydrogenase; WBC, white blood cells; ICU, intensive care unit; ARDS, acute respiratory distress syndrome.**

| **Clinical characteristics and outcomes** | **All** | **Antigenemia (NO)** | **Antigenemia (YES)** | ***p* value** |
|---|---|---|---|---|
| No. | 400 | 187 | 213 | - |
| | | | | |
| Age (years) | 72.0 (24.0) | 71.0 (23.0) | 74.0 (26.0) | 0.300 |
| Male | 204 (51.0) | 94 (50.3) | 110 (51.6) | 0.780 |
| Current smoker | 15 (3.7) | 10 (5.3) | 5(2.3) | 0.115 |

| **Comorbidities** | | | | |
|---|---|---|---|---|
| Hypertension | 210 (52.5) | 92 (49.2) | 118 (55.4) | 0.215 |
| Dyslipidemia | 139 (34.7) | 60 (32.1) | 79 (37.1) | 0.294 |
| Diabetes | 98 (24.5) | 41 (21.9) | 57 (26.8) | 0.262 |
| Obesity | 88 (22.0) | 37 (19.8) | 51 (23.9) | 0.317 |
| Ischemic heart disease | 40 (10.0) | 14 (7.5) | 26 (12.2) | 0.116 |
| Chronic heart failure | 32 (8.0) | 9 (4.8) | 23 (10.8) | 0.028 |
| Stroke | 21 (5.2) | 9 (4.8) | 12 (5.6) | 0.713 |
| Central Nervous System disease (Other than stroke) | 83 (20.7) | 44 (23.5) | 39 (18.3) | 0.199 |
| Atrial fibrillation | 45 (11.2) | 19 (10.2) | 26 (12.2) | 0.518 |
| Chronic renal disease | 50 (12.5) | 18 (9.6) | 32 (15.0) | 0.103 |
| Asthma | 21 (5.2) | 5 (2.7) | 16 (7.5) | **0.030** |
| COPD | 42 (10.5) | 17 (9.1) | 25 (11.7) | 0.389 |
| Cancer | 37 (9.2) | 16 (8.6) | 21 (9.9) | 0.654 |
| Chronic inflammatory bowel disease | 4 (1.0) | 2 (1.1) | 2 (0.9) | 0.896 |
| Chronic hepatic disease | 12 (30) | 7 (3.7) | 5 (2.3) | 0.592 |
| Congenital thrombophilia | 3 (0.7) | 2 (1.1) | 1 (0.5) | 0.488 |
| Apnea-hypopnea syndrome | 18 (4.5) | 10 (5.3) | 8 (3.8) | 0.440 |
| Charlson score* (393) | 4.0 (3.0) | 3.5 (3.0) | 4.0 (4.0) | 0.312 |

| **At hospital admission** | | | | |
|---|---|---|---|---|
| Asthenia *(397) | 203 (50.7) | 103 (55.1) | 100 (47.6) | 0.138 |
| Anorexia * (392) | 39 (9.7) | 22 (12.0) | 17 (8.1) | 0.199 |
| Pharyngeal pain | 10 (2.5) | 9 (4.8) | 1 (0.5) | **0.006** |
| Odynophagia* (397) | 18 (4.5) | 13 (7.0) | 5 (2.4) | **0.027** |
| Conjunctivitis* (398) | 1 (0.2) | 1 (0.5) | 0 | 0.288 |
| Cough* (398) | 210 (52.5) | 91 (48.9) | 119 (56.1) | 0.151 |
| Dyspnoea* (399) | 242 (60.5) | 106 (57.0) | 136 (63.8) | 0.162 |
| Pleuritic pain* (394) | 33 (8.2) | 14 (7.6) | 19 (9.0) | 0.607 |
| Sputum | 50 (12.5) | 23 (12.3) | 27 (12.7) | 0.910 |
| Arthralgia* (399) | 16 (4.0) | 4 (2.2) | 12 (5.6) | 0.077 |
| Myalgia* (399) | 62 (15.5) | 28 (15.1) | 34 (16.0) | 0.803 |
| Nausea, vomiting* (398) | 49 (12.2) | 27 (14.6) | 22 (103) | 0.196 |
| Diarrhea* (397) | 83 (20.7) | 36 (19.6) | 47 (22.1) | 0.541 |
| Abdominal pain* (398) | 22 (5.5) | 13 (7.0) | 9 (4.2) | 0.222 |
| Anosmia | 41 (10.2) | 15 (8.0) | 26 (12.2) | 0.169 |
| Dysgeusia | 43 (10.7) | 20 (10.7) | 23 (10.8) | 0.974 |
| Headache | 33 (8.2) | 16 (8.6) | 17 (8.0) | 0.835 |
| Confusion | 22 (5.5) | 11 (5.9) | 11 (5.2) | 0.752 |
| Agitation | 8 (2.0) | 4 (2.1) | 4 (1.9) | 0.852 |
| Vertigo | 6 (1.5) | 1 (0.5) | 5(2.3) | 0.137 |
| Ataxia | 1 (1.5) | 0 | 1 (0.5) | 0.348 |
| Depressed level of consciousness | 2.5 (6.2) | 12 (6.4) | 13 (6.1) | 0.897 |
| Paresthesia | 1 (0.2) | 1 (0.5) | 0 | 0.285 |
| Neuropathic pain | 1 (0.2) | 1 (0.5) | 0 | 0.285 |
| Skin lesions | 7 (0.2) | 4 (2.1) | 3 (1.4) | 0.578 |
| Coinfection | 24 (6.0) | 12 (6.4) | 12 (5.6) | 0.742 |

| **Measurements at hospital admission** | | | | |
|---|---|---|---|---|
| Heart rate (beats per min)* (398) | 87.0 (24.0) | 87.0 (23.0) | 87.0 (25.0) | 0.722 |
| Systolic blood pressure (mmHg)* (398) | 130.0 (30.0) | 130.50 (28.2) | 130.0 (31.0) | 0.835 |
| Diastolic blood pressure (mmHg)* (398) | 730 (17.0) | 74.0 (15.0) | 73.0 (19.0) | 0.607 |
| MAP* (398) | 93.3 (18.1) | 93.8 (15.7) | 92.5 (22.0) | 0.728 |
| Temperature (°C)* (380) | 36.6 (1.3) | 36.4 (0.9) | 36.8 (1.4) | **< 0.001** |
| % of patients with respiratory rate > 30 (breaths per min)* (368) | 76 (20.6) | 24 (13.6) | 52 (27.1) | **0.001** |
| Oxygen saturation (%)* (394) | 95.0 (5.0) | 95.0 (5.0) | 94.0 (6.0) | 0.109 |
| PaO₂/FIO₂ * (396) | 338.0 (172.0) | 376.0 (205.0) | 328.0 (128.0) | **0.024** |
| Bilateral pulmonary infiltrate [n (%)] | 248 (62.0) | 108 (57.8) | 140 (65.7) | 0.101 |
| Bilirrubin (mg/dL) | 0.4 (0.3) | 0.5 (0.3) | 0.4 (0.2) | **0.001** |
| Creatinine (mg/dL) | 0.8 (0.3) | 0.8 (0.4) | 0.9 (0.4) | **0.002** |
| BUN (mg/dL)* (382) | 21.0 (15.5) | 19.0 (13.9) | 22.0 (17.3) | 0.058 |
| GPT (UI/L)* (378) | 25.0 (24.0) | 26.0 (32.0) | 24.0 (20.0) | 0.266 |
| Na (mmol/L)* (388) | 139.6 (5.0) | 139.7 (4.5) | 139.4 (5.0) | 0.548 |
| K (mmol/L)* (387) | 4.1 (0.6) | 4.2 (0.6) | 4.0 (0.7) | **0.022** |
| C-reactive protein (mg/L)* (372) | 78.0 (95.0) | 63.0 (95.0) | 90.0 (87.0) | **0.002** |
| LDH (UI/L)* (315) | 392.0 (367.0) | 363.0 (311.0) | 400.5 (372.0) | **0.050** |
| Hemoglobin (g/dL)* (392) | 13.3 (2.5) | 13.2 (2.2) | 13.2 (2.6) | 0.473 |
| Mean Corpuscular Volume (fL per cell)* (394) | 89.0 (7.3) | 88.8 (6.7) | 89.3 (7.4) | 0.180 |
| Mean corpuscular hemoglobin (pg per cell) * (394) | 30.0 (2.5) | 30.1 (2.8) | 29.9 (2.4) | 0.513 |
| WBC (cells/mm3) * (394) | 6100.0 (4127.5) | 6240.0 (4610.0) | 5680.0 (3975.0) | 0.124 |
| Lymphocytes (cells/mm3) * (394) | 900.0 (665.0) | 1000.0 (770.0) | 800.0 (605.0) | **< 0.001** |
| Neutrophils (cells/mm3) * (394) | 4305.0 (3807.5) | 4450.0 (4140.0) | 4300.0 (3800.0) | 0.613 |
| Monocytes (cells/mm3) * (394) | 400.0 (340.0) | 500.0 (380.0) | 370.0 (300.0) | **< 0.001** |
| Platelets (cell x 103/ µl) | 187.0 (109.7) | 219.0 (134.0) | 167.0 (84.5) | **< 0.001** |

| **Severity Scores** | | | | |
|---|---|---|---|---|
| SOFA * (394) | 2.0 (1.0) | 1.0 (1.0) | 2.0 (2.0) | **< 0.001** |
| SOFA (respiration) > 0 * (399) | 247 (61.9) | 101 (54.6) | 146 (69.2) | **0.003** |
| SOFA (coagulation) > 0 | 111 (27.7) | 34 (18.2) | 77 (36.2) | **< 0.001** |
| SOFA (renal) > 0 | 73 (18.2) | 30 (16.0) | 43 (20.2) | 0.284 |
| SOFA (liver) > 0 | 11 (2.7) | 6 (3.2) | 5 (2.3) | 0.599 |
| SOFA (cardiovascular) > 0 * (398) | 27 (6.8) | 11 (5.9) | 16 (7.5) | 0.518 |
| SOFA (CNS) > 0 | 22 (5.5) | 11 (5.9) | 11 (5.2) | 0.753 |
| Sepsis* (394) | 213 (54.1) | 84 (45.7) | 129 (61.4) | **0.002** |
| CURB-65 * (396) | 2.0 (20) | 1.0 (1.0) | 2.0 (2.0) | **0.009** |
| MulBTSA *(397) | 9.0 (6.0) | 8.0 (7.0) | 9.0 (5.0) | **0.037** |

| **Treatments and outcomes** | | | | |
|---|---|---|---|---|
| Day since symptoms onset to hospital admission (days) * (378) | 5.0 (5.0) | 5.0 (6.0) | 4.0 (5.0) | 0.131 |
| Hospital stay (days) | 8.0 (8.0) | 6.0 (5.0) | 10.0 (10.0) | **< 0.001** |
| ICU admission | 39 (9.7) | 12 (6.4) | 27 (12.7) | **0.035** |
| Low-flow oxygen therapy | 324 (81.0) | 133 (71.1) | 191 (89.7) | **< 0.001** |
| Non-invasive mechanical ventilation | 36 (9.0) | 9 (4.8) | 27 (12.7) | **0.006** |
| High-flow oxygen therapy | 83 (20.7) | 26 (13.9) | 57 (26.8) | **0.002** |
| Invasive mechanical ventilation | 23 (5.7) | 6 (3.2) | 17 (8.0) | **0.041** |
| Prone Position* (399) | 23 (5.8) | 7 (3.8) | 16 (7.5) | 0.109 |
| Hydroxychloroquine* (397) | 1 (0.2) | 0 | 1 (0.5) | 0.345 |
| Lopinavir/ritonavir* (399) | 3 (0.7) | 1 (0.5) | 2 (0.9) | 0.637 |
| Remdesivir* (399) | 51 (12.8) | 21 (11.2) | 30 (14.2) | 0.383 |
| Corticoids* (398) | 278 (69.8) | 108 (58.4) | 170 (79.8) | **< 0.001** |
| Tocilizumab* (398) | 98 (24.6) | 31 (16.6) | 67 (31.8) | **< 0.001** |
| Azithromycin* (399) | 154 (38.5) | 71 (38.0) | 83 (39.2) | 0.809 |
| Other antibiotics | 247 (61.7) | 108 (57.8) | 139 (65.3) | 0.123 |
| Convalescent plasma* (399) | 2 (0.5) | 1 (0.5) | 1 (0.5) | 0.929 |
| Heart failure | 3 (0.7) | 2 (1.1) | 1 (0.5) | 0.488 |
| Acute kidney injury | 6 (1.5) | 3 (1.6) | 3 (1.4) | 0.872 |
| Stroke* (399) | 1 (0.2) | 0 | 1 (0.5) | 0.347 |
| Pulmonary thromboembolism* (399) | 12 (3.0) | 8 (4.3) | 4 (1.9) | 0.163 |
| Deep vein thrombosis* (399) | 1 (0.2) | 0 | 1 (0.5) | 0.347 |
| Acute coronary syndrome* (399) | 3 (0.7) | 1 (0.5) | 2 (0.9) | 0.637 |
| Arrhythmias* (399) | 15 (3.8) | 5 (2.7) | 10 (4.7) | 0.284 |
| Hemophagocitic syndrome* (399) | 2 (0.5) | 1 (0.5) | 1 (0.5) | 0.929 |
| ARDS* (399) | 138 (34.6) | 52 (27.8) | 86 (40.6) | **0.008** |
| Confusion* (388) | 45 (11.5) | 16 (8.9) | 29 (13.9) | 0.130 |
| Secondary hospital-acquired infections* (397) | 19 (4.8) | 6 (6.0) | 13 (6.1) | 0.179 |
| Bleeding* (399) | 16 (40) | 7 (3.7) | 9 (4.2) | 0.799 |
| 90-Day Mortality | 78 (19.5) | 232 (12.3) | 55 (25.8) | **0.001** |

### Table 3

**Table 3. Multivariate logistic regression analysis to evaluate the association between N-antigenemia and sepsis at hospital admission.**

| | **OR** | **CI95%** | | ***p*** | **OR** | **CI95%** | | ***p*** |
|---|---|---|---|---|---|---|---|---|
| Age (years) | 1.0 | 1.0 | 1.0 | 0.372 | 1.0 | 1.0 | 1.0 | 0.436 |
| Sex (male) | 1.7 | 1.1 | 2.6 | 0.022 | 1.6 | 1.0 | 2.5 | 0.051 |
| Hypertension | 1.2 | 0.7 | 1.9 | 0.554 | 1.1 | 0.7 | 1.9 | 0.597 |
| Diabetes | 1.2 | 0.7 | 2.2 | 0.456 | 1.2 | 0.7 | 2.2 | 0.462 |
| Obesity | 1.5 | 0.8 | 2.5 | 0.172 | 1.4 | 0.8 | 2.5 | 0.214 |
| Chronic heart failure | 1.6 | 0.6 | 4.0 | 0.355 | 1.7 | 0.6 | 4.3 | 0.292 |
| Chronic renal failure | 4.9 | 2.0 | 11.7 | 0.000 | 4.3 | 1.8 | 10.5 | 0.001 |
| COPD | 2.6 | 1.2 | 5.8 | 0.017 | 2.7 | 1.2 | 5.9 | 0.015 |
| Neoplasia (active) | 2.4 | 1.1 | 5.4 | 0.033 | 2.6 | 1.1 | 5.8 | 0.023 |
| Bilateral pneumonia in the Chest X-ray | 1.5 | 0.9 | 2.3 | 0.099 | 1.3 | 0.8 | 2.1 | 0.216 |
| **ANTIGENEMIA (Panbio^{®}) (YES)** | **1.6** | **1.1** | **2.5** | **0.023** | | | | |
| **ANTIGENEMIA (COV-QUANTO^{®}) >575 pg/mL** | | | | | **2.2** | **1.4** | **3.4** | **0.001** |

### Table 4

**Table 4. Multivariate logistic regression analysis to evaluate the antigenemia as predictor of 90-day mortality.**

| | **OR** | **CI95%** | | ***p*** | **OR** | **CI95%** | | ***p*** |
|---|---|---|---|---|---|---|---|---|
| Age (years) | 1.1 | 1.0 | 1.1 | 0.000 | 1.1 | 1.0 | 1.1 | 0.000 |
| Hypertesion | 1.2 | 0.6 | 2.3 | 0.601 | 1.2 | 0.6 | 2.2 | 0.648 |
| Obesity | 0.6 | 0.3 | 1.5 | 0.283 | 0.7 | 0.3 | 1.5 | 0.315 |
| Ischemic cardiopathy | 0.8 | 0.3 | 2.0 | 0.663 | 0.9 | 0.4 | 2.2 | 0.889 |
| Chronic heart failure | 1.4 | 0.5 | 3.6 | 0.480 | 1.5 | 0.6 | 3.9 | 0.378 |
| Chronic disease of the CNS (other than stroke) | 2.0 | 1.0 | 4.0 | 0.041 | 1.8 | 0.9 | 3.6 | 0.076 |
| Atrial fibrillation | 1.0 | 0.4 | 2.2 | 0.935 | 0.9 | 0.4 | 2.1 | 0.826 |
| Chronic Renal Disease | 0.9 | 0.4 | 2.1 | 0.773 | 0.8 | 0.4 | 2.0 | 0.677 |
| Co-infection at admission | 1.6 | 0.5 | 4.6 | 0.426 | 1.5 | 0.5 | 4.4 | 0.449 |
| Arrhythmia during hospitalzation | 1.6 | 0.5 | 5.8 | 0.463 | 1.8 | 0.5 | 6.7 | 0.352 |
| Remdesivir | 0.9 | 0.2 | 3.0 | 0.804 | 0.8 | 0.2 | 2.9 | 0.771 |
| Mechanical ventilation | 6.8 | 2.1 | 22.2 | 0.002 | 6.3 | 1.9 | 21.3 | 0.003 |
| SOFA | 1.4 | 1.1 | 1.8 | 0.002 | 1.4 | 1.1 | 1.8 | 0.002 |
| **ANTIGENEMIA (Panbio^{®}) (YES)** | **2.2** | **1.2** | **4.1** | **0.015** | | | | |
| **ANTIGENEMIA (COV-QUANTO^{®}) >1835 pg/mL** | | | | | **1.9** | **1.0** | **3.5** | **0.043** |

## Claims

1. *In vitro* method for the prognosis of mortality risk in patients suffering from SARS-CoV-2 infection, which comprises determining the presence or the level of SARS-CoV-2 antigen nucleocapsid (N) in plasma, serum or blood samples obtained from the patient, wherein the determination of the presence of SARS-CoV-2 antigen nucleocapsid (N), or the quantification of a level of SARS-CoV-2 antigen nucleocapsid (N) statistically higher as compared with a pre-established threshold value, is an indication of bad prognosis and mortality risk.

2. *In vitro* method for predicting the response of patients suffering from SARS-CoV-2 infection to the antiviral remdesivir which comprises assessing the presence or the level of SARS-CoV-2 antigen nucleocapsid (N) in plasma, serum or blood samples obtained from the patient, wherein if the presence of SARS-CoV-2 antigen nucleocapsid (N) is identified, or a statistically higher level of SARS-CoV-2 antigen nucleocapsid (N) is quantified as compared with a pre-established threshold value, this is an indication that the patient may respond to the antiviral remdesivir.

3. *In vitro* method for selecting patients suffering from SARS-CoV-2 infection for receiving the antiviral remdesivir, which comprises determining the presence or the level of SARS-CoV-2 antigen nucleocapsid (N) in plasma, serum or blood samples obtained from the patient, wherein if the presence of SARS-CoV-2 antigen nucleocapsid (N) is identified, or a statistically higher level of SARS-CoV-2 antigen nucleocapsid (N) is quantified as compared with a pre-established threshold value, the patient is elected for receiving the antiviral remdesivir.

4. *In vitro* method, according to any of the previous claims, wherein the patient is suffering from sepsis secondary to SARS-CoV-2 infection.

5. *In vitro* method, according to any of the previous claims, **characterized in that** the presence or the level of the antigen is measured by using mass spectrometry or an immunoassay, preferably ELISA, immunochromatography, nephelometry, Luminex, SimplePlex, or any other technique based on the antigen-antibody reaction, and also those based on recognition of SARS-CoV-2 antigens by DNA or RNA molecules such as those using aptamers or oligonucleotide-labelled antibodies.

6. *In vitro* use of SARS-CoV-2 antigen nucleocapsid (N) in plasma, serum or blood samples obtained from the patient, for the prognosis of the mortality risk of patients suffering from SARS-CoV-2 infection, for predicting the response of patients suffering from SARS-CoV-2 infection to the antiviral remdesivir or for selecting patients suffering from SARS-CoV-2 infection for receiving the antiviral remdesivir.

## Patentansprüche

1. *In-vitro*-Verfahren für die Prognose des Sterblichkeitsrisikos in Patienten, welche unter einer SARS-CoV-2-Infektion leiden, welches das Bestimmen des Vorhandenseins oder des Niveaus von SARS-CoV-2-Antigen-Nucleocapsid (N) in Plasma-, Serum- oder Blutproben erhalten aus dem Patienten umfasst, wobei die Bestimmung des Vorhandenseins von SARS-CoV-2-Antigen-Nucleocapsid (N), oder die Quantifizierung eines statistisch höheren Niveaus von SARS-CoV-2-Antigen-Nucleocapsid (N) im Vergleich zu einem vorher festgesetzten Schwellenwert, ein Hinweis von schlechter Prognose und Sterblichkeitsrisiko ist.

2. *In-vitro*-Verfahren zur Vorhersage der Reaktion von Patienten, welche unter einer SARS-CoV-2-Infektion leiden, auf das antivirale Medikament Remdesivir, welches das Beurteilen des Vorhandenseins oder des Niveaus von SARS-CoV-2-Antigen-Nucleocapsid (N) in Plasma-, Serum- oder Blutproben erhalten aus dem Patienten umfasst, wobei, wenn das Vorhandensein von SARS-CoV-2-Antigen-Nucleocapsid (N) identifiziert wird, oder ein statistisch höheres Niveau von SARS-CoV-2-Antigen-Nucleocapsid (N) im Vergleich zu einem vorher festgesetzten Schwellenwert quantifiziert wird, dies ein Hinweis davon ist, dass der Patient auf das antivirale Medikament Remdesivir reagieren kann.

3. *In-vitro*-Verfahren zur Auswahl von Patienten, welche unter einer SARS-CoV-2-Infektion leiden, um das antivirale Medikament Remdesivir zu bekommen, welches das Bestimmen des Vorhandenseins oder des Niveaus von SARS-CoV-2-Antigen-Nucleocapsid (N) in Plasma-, Serum- oder Blutproben erhalten aus dem Patienten umfasst, wobei, wenn das Vorhandensein von SARS-CoV-2-Antigen-Nucleocapsid (N) identifiziert wird, oder ein statistisch höheres Niveau von SARS-CoV-2-Antigen-Nucleocapsid (N) im Vergleich zu einem vorher festgesetzten Schwellenwert quantifiziert wird, der Patient gewählt wird, um das antivirale Medikament Remdesivir zu bekommen.

4. *In-vitro*-Verfahren nach einem der vorhergehenden Ansprüche, wobei der Patient unter Sepsis sekundär zu einer SARS-CoV-2-Infektion leidet.

5. *In-vitro*-Verfahren nach einem der vorhergehenden Ansprüche, **dadurch gekennzeichnet, dass** das Vorhandensein oder das Niveau des Antigens unter Verwendung von Massenspektrometrie oder einem Immuntest, vorzugsweise ELISA, Immunchromatographie, Nephelometrie, Luminex, SimplePlex oder irgendeiner anderen Technik basierend auf der Antigen-Antikörper-Reaktion, und auch diejenigen basierend auf der Erkennung von SARS-CoV-2-Antigenen mittels DNA- oder RNA-Moleküle, wie diejenigen, welche Aptamere oder mit Oligonukleotiden-markierte Antikörper verwenden, gemessen wird.

6. *In-vitro*-Verwendung von SARS-CoV-2-Antigen-Nucleocapsid (N) in Plasma-, Serum- oder Blutproben erhalten aus dem Patienten, für die Prognose des Sterblichkeitsrisikos von Patienten, welche unter einer SARS-CoV-2-Infektion leiden, zur Vorhersage der Reaktion von Patienten, welche unter einer SARS-CoV-2-Infektion leiden, auf das antivirale Medikament Remdesivir oder zur Auswahl von Patienten, welche unter einer SARS-CoV-2-Infektion leiden, um das antivirale Medikament Remdesivir zu bekommen.

## Revendications

1. Procédé *in vitro* pour le pronostic du risque de mortalité chez des patients atteints d'une infection par SARS-CoV-2, qui comprend la détermination de la présence ou le niveau de nucléocapside (N) d'antigène de SARS-CoV-2 dans des échantillons de plasma, de sérum ou de sang obtenus du patient, dans lequel la détermination de la présence de nucléocapside (N) d'antigène de SARS-CoV-2, ou la quantification d'un niveau de nucléocapside (N) d'antigène de SARS-CoV-2 statistiquement supérieur par rapport à une valeur seuil préétablie, est une indication d'un mauvais pronostic et du risque de mortalité.

2. Procédé *in vitro* pour prédire la réponse de patients atteints d'une infection par SARS-CoV-2 au composé antiviral remdesivir qui comprend l'évaluation de la présence ou le niveau de nucléocapside (N) d'antigène de SARS-CoV-2 dans des échantillons de plasma, de sérum ou de sang obtenus du patient, dans lequel, si la présence de nucléocapside (N) d'antigène de SARS-CoV-2 est identifiée, ou si un niveau statistiquement supérieur de nucléocapside (N) d'antigène de SARS-CoV-2 est quantifié par rapport à une valeur seuil préétablie, ceci est une indication que le patient peut répondre au composé antiviral remdesivir.

3. Procédé *in vitro* pour sélectionner des patients atteints d'une infection par SARS-CoV-2 pour recevoir le composé antiviral remdesivir, qui comprend la détermination de la présence ou le niveau de nucléocapside (N) d'antigène de SARS-CoV-2 dans des échantillons de plasma, de sérum ou de sang obtenus du patient, dans lequel, si la présence de nucléocapside (N) d'antigène de SARS-CoV-2 est identifiée, ou si un niveau statistiquement supérieur de nucléocapside (N) d'antigène de SARS-CoV-2 est quantifié par rapport à une valeur seuil préétablie, le patient est choisi pour recevoir le composé antiviral remdesivir.

4. Procédé *in vitro,* selon l'une quelconque des revendications précédentes, dans lequel le patient est atteint d'un sepsis secondaire à une infection par SARS-CoV-2.

5. Procédé *in vitro,* selon l'une quelconque des revendications précédentes, **caractérisé en ce que** la présence ou le niveau de l'antigène est mesuré par le biais de spectrométrie de masse ou un immunoessai, de préférence ELISA, immunochromatographie, néphélométrie, Luminex, SimplePlex, ou toute autre technique basée sur la réaction antigène-anticorps, et également celles basées sur la reconnaissance des antigènes de SARS-CoV-2 par des molécules d'ADN ou d'ARN telles que celles utilisant des aptamères ou des anticorps marqués par des oligonucléotides.

6. Utilisation *in vitro* de nucléocapside (N) d'antigène de SARS-CoV-2 dans des échantillons de plasma, de sérum ou de sang obtenus du patient, pour le pronostic du risque de mortalité de patients atteints d'une infection par SARS-CoV-2, pour prédire la réponse de patients atteints d'une infection par SARS-CoV-2 au composé antiviral remdesivir ou pour sélectionner des patients atteints d'une infection par SARS-CoV-2 pour recevoir le composé antiviral remdesivir.
